# EUROPEAN PATENT APPLICATION

(11) **EP 4 714 474 A1**
(43) Date of publication of application: **25.03.2026**
(21) Application number: 25197062.0
(22) Date of filing: 20.08.2025
(51) Int. Cl.: A61M 1/02, A61M 1/36, A61M 1/38

(54) **BEDSIDE CELL PROCESSING SYSTEM AND METHOD HAVING A REINFUSION SYSTEM**

(30) Priority: 21.08.2024 US 202463685414 P; 19.08.2025 US 202519304521
(71) Applicant: Fenwal, Inc., Lake Zurich, IL 60047 (US)
(72) Inventor: WEGENER, Christopher, Lake Zurich, 60047 (US); MAIBAUER, John, Lake Zurich, 60047 (US)
(74) Representative: Maikowski & Ninnemann Patentanwälte Partnerschaft mbB

(57) **Abstract**

Systems and method for separating, collecting, treating target cells and infusing the treated target cells to a patient are disclosed. The closed system includes a predefined fluid pathway providing flow communication between a whole blood source and said patient.

## Description

### Field of the Disclosure

The present disclosure is directed to the processing and collection of blood and its components. More particularly, the present disclosure is generally directed to apparatus, systems and methods for processing or treating cells from previously collected blood and returning the treated cells to a bedside patient. The present disclosure is further directed to an apparatus, systems and methods for processing or treating cells that does not involve apheresis, and directly processes previously collected whole blood.

### Background

U.S. Patent Application Serial No.: 18/646,247, filed April 25, 2024, the contents of which are hereby incorporated by reference, discloses a processing system that separates and collects particular target cells and treats the target cells collected from a patient. The system utilizes an apheresis system and a reinfusion system whereby the processed cells are returned to the patient via the apheresis system/reinfusion system. The system described in U.S. Patent Application Serial No.: 18/646,247 may include integrated apheresis and reinfusion flow paths with either single needle or dual needle patient access.

As an alternative to the apheresis-based systems and methods described in U.S. Patent Application Serial No.:18/646,247, it would be desirable to provide a simplified system and method whereby whole blood previously collected in a container from a patient (or donor) can be processed and the target cells treated and (re)infused to a patient, all in a pre-defined and continuous fluid circuit, preferably without disconnection of any part of the circuit.

### Summary

In one aspect, the present disclosure is directed to a bedside cell processing system. The system includes a source of previously collected whole blood and a cell processing device configured to receive the target cells from the whole blood source and to separate target cells from the whole blood and further process the target cells. The system further includes a reinfusion system configured to receive the processed target cells from the cell processing system and to return the processed cells to a patient. A pre-defined fluid pathway providing flow communication between said whole blood source and said patient is also included.

In another aspect, a cell processing method is provided. The method includes connecting a container for receiving whole blood to an inlet of a cell processing device and connecting a treated target cell product container to an outlet of the cell processing device. Whole blood from the container is then introduced into the cell processing device. The whole blood is separated into target cells and other blood components in the cell processing device. The method further includes treating the target cells within the cell processing device and then introducing the treated target cells into the target cell product container. Thus, according to this aspect an ex-vivo method is provided wherein said method is directed to a treatment of cells separated from whole blood and subsequently introducing and storing said treated cells in a suitable container.

The systems and methods of the present disclosure utilize a continuous, pre-defined fluid circuit between the blood source (container) and the patient who receives the autologous and treated target cells. Preferably, the patient receives the treated target cells directly from the target cell product container without the need for disconnecting the container from the circuit.

### Brief Description of the Drawings

Figure 1 is a schematic diagram of a bedside cell processing system in accordance with the present disclosure;
Figure 2 is schematic diagram of an example cell processing and reinfusion system for use in the bedside cell processing system shown in Figure 1 with manual reinfusion of the treated target cells;
Figure 3 is schematic diagram of an example of a cell processing and reinfusion system for use in the bedside cell processing system shown in Figure 1 with automated reinfusion of the treated target cells; and
Figure 4 is a flow chart of the blood processing method in accordance with the present disclosure.

### Detailed Description of the Embodiments

The embodiments disclosed herein are for the purpose of providing an exemplary description of the present subject matter. They are, however, only exemplary, and the present subject matter may be embodied in various forms. Therefore, specific details disclosed herein are not to be interpreted as limiting the subject matter as defined in the accompanying claims.

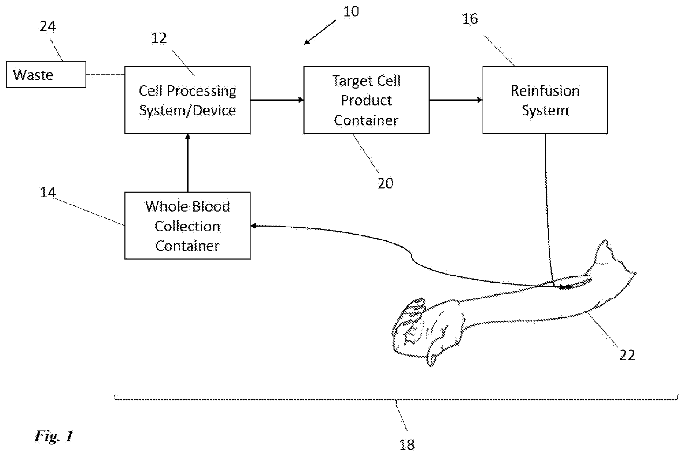

Fig. 1 is a schematic diagram depicting the general configuration of the bedside blood processing system 10 described below. As shown in Fig. 1, bedside blood processing system 10 includes a cell processing device or system 12 and a collection container of previously collected whole blood 14 in flow communication with device/system 12. Container 14 is preferably preconnected to cell processing device/system 12 as an empty container that receives blood from the patient, or a filled container of whole blood that was previously collected from the patient and is sterile docked to the cell processing device/system 12 at the time of use. Filled container 14 may also be accessed by a spiked connection to a port on the container.

As further shown in Fig. 1, the cell processing device/system 12 is in fluid communication with target cell product container 20 which, like whole blood collection container 14, is preconnected to an outlet line of the cell processing device/system 12. Target cell product container 20 receives target cells that have been separated from the patient's whole blood and have been treated in the cell processing device or system 12. Target cell product container 20 may be part of and/or in flow communication with a reinfusion system 16 for reinfusing the treated and autologous and now treated target cells to the patient 22 and may be preconnected to the reinfusion system 16 at the time of manufacture of the bedside blood processing system 10. In accordance with the present disclosure and as shown in Fig. 1, target cell product container is also in direct flow communication with patient 11. As such, a predefined and closed fluid path from the target cell product container to the patient during reinfusion may beprovided. This arrangement eliminates the need to detach target cell product container from the remainder of the kit to perform the reinfusion. A waste container 24 may be included for receiving non-targeted cells and blood components.

Thus, the bedside blood processing system 10 described above and generally depicted in Fig. 1 provides a continuous and pre-defined fluid pathway 18 that provides a closed system, maintains sterility and does not require disconnection of any of its parts.

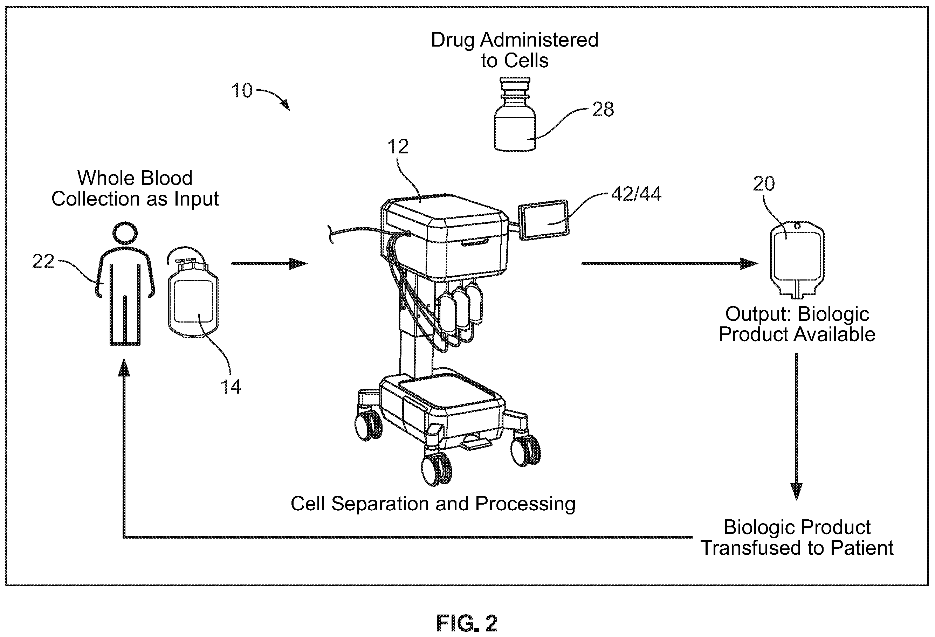

Turning now to Fig. 2, there is shown a more specific embodiment of the bedside cell processing system 10. As shown in Fig. 2, a container 14 of previously collected whole blood obtained from a patient 22 is attached to a cell separation and processing device 12. The cell separation and processing device 12 may be any device that is configured to separate the whole blood into its constituent components such as red blood cells, white blood cells (leukocytes), stem cells, platelets, plasma and collect a desired target cell. The separation may be achieved by introducing the whole blood into a centrifuge, a spinning membrane or a microfluidic separation module. Alternatively, the separation may be a passive separation as will be described in more detail below. The cell separation and processing device may also be configured to "process" the separated target cells. By "process" or "processing" it is meant that the target cells are treated by an agent such as a liquid agent, a drug, light, affinity beads, incubation to alter or modify the properties of the target cell. As shown in Fig. 2, a drug or agent delivery system (represented by vial 28) may be incorporated into the cell processing system/device 12. Vial 28 or other drug dispensing device may be pre-connected within the cell processing device/system 12 as part of the pre-defined fluid circuit. System/device 12 includes a controller (not shown) including a microprocessor and memory for directing the operation of the device/system 12 including cell collection, cell separation, drug delivery from container/vial 28 via pumps or other means and, optionally, reinfusion. Cell separation/and processing device 12 may include an input 42/output 44 device such as a graphical user interface for entering parameters of the blood cell processing protocol and for receiving information regarding the status of the cell processing.

One example of a cell separation and processing device is described in International Patent Application No. PCT/US2024/015424 and International Patent Application No. PCT/US2024/015404, the contents of which are incorporated herein by reference. The cell separation and processing device described in International Patent Application No. PCT/US2024/015424 and International Patent Application No. PCT/US2024/015404 is a modular device which includes integrated or otherwise associated modules for cell sorting, cell concentration, cell selection and, optionally, cargo delivery. In the particular system of PCT/US2024/015424, cell separation and processing may occur on a microfluidic scale, although it will be understood the any type of cell separation and processing device may be used with the bedside blood processing system described herein.

Once the target cells have been treated in cell separation and processing device 12, they are collected in target cell product container 20. As shown in Fig. 2, the treated target cells may be (re)infused to the patient 22 via venipuncture made at the time of reinfusion. Ideally, target cell product container is not disconnected from the system or the continuous circuit. In one embodiment, reinfusion of the treated target cells may be by simple gravity drain of the treated target cells into the patient. Target cell container may include a tube defining a flow path and terminating in a venipuncture needle to allow for infusion of the treated target cells.

As further shown in Fig. 1, whole blood withdrawal and treated target cell reinfusion may be carried out using a single-needle fluid circuit i.e., where whole blood is withdrawn and treated target cells are returned through the same venipuncture needle. Alternatively, the circuit may be of a dual needle configuration wherein the initial blood draw into preconnected whole blood collection container 14 occurs via a first venipuncture and reinfusion of the treated target cells occurs via a second venipuncture at the time of the reinfusion.

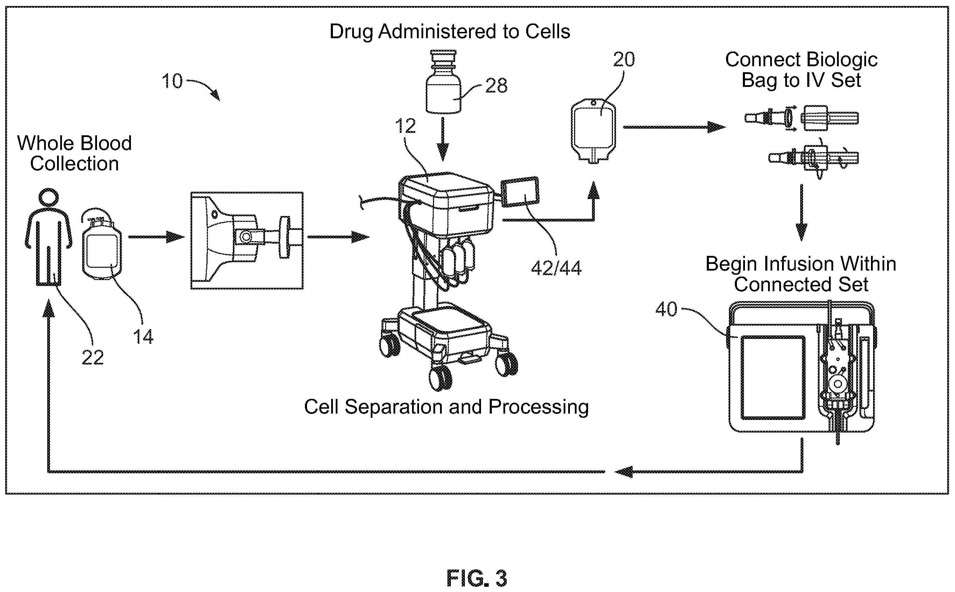

An alternative bedside cell processing system is shown in Fig. 3 (which in many respects is identical to the system shown in Fig. 2.) In the system of Fig. 3, the whole blood container (with previously collected whole blood), is connected to system 10 at the time of use. Whole blood container 14 includes a sealed port that may be accessed by a piercing member that is connected to the fluid circuit of cell processing system/device 12 to make a sterile connection. Once the sterile connection is made, cell separation and processing may proceed as described in connection with the system of Fig. 2.

In the system of Fig. 3, target cell product container 20 may be connected to infusion pump 40-the container 20 and pump 40 comprising the reinfusion system 16 shown in Fig. 1. Container 40 may be preconnected to infusion pump 40 or connected to infusion pump 40 at the time of reinfusion via a sterile connection of the type that will be known to those of skill in the art, such as, but not limited to, a needleless connection. Once the desired amount of treated target cells in target cell product container is reached, infusion pump 40 will deliver (infuse) the treated cells to the connected patient 22. Reinfusion system 16 and more particularly, infusion pump 40 may be operated by the controller associated with the cell processing device or system 12 described above, or may include its own dedicated controller configured to initiate reinfusion at a predetermined time .

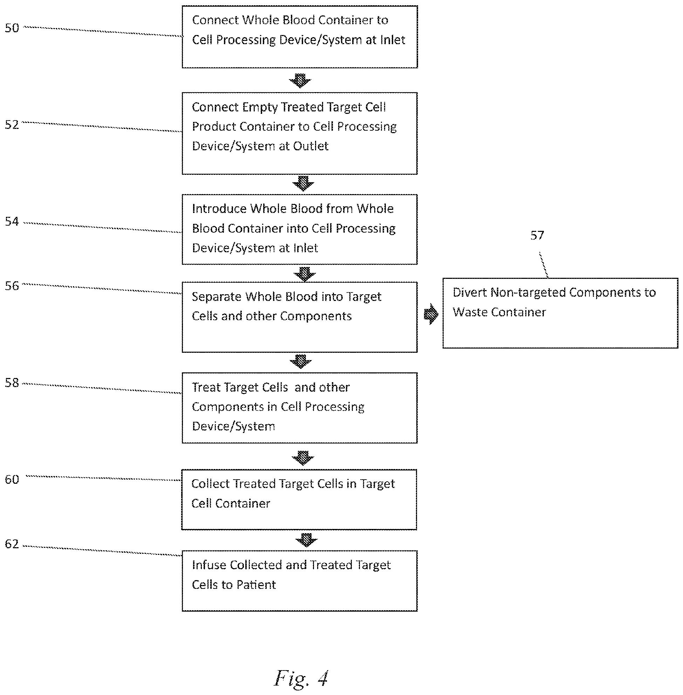

Fig. 4 shows a method for establishing and using the bedside cell processing system 10 including reinfusion system, in accordance with the present disclosure. A central aspect of the present method is the establishment of a pre-defined fluid circuit. Accordingly, in a first step 50, a container for holding a volume of whole blood is connected to a cell processing device/system. The container may be empty and preconnected to the fluid circuit of the cell processing device/system at the time of manufacture. At the time of processing, using a venipuncture pre-attached to the whole blood container, whole blood may drawn from the patient and allowed to drain into container 14. Alternatively, the container may be filled with whole blood from an earlier collection from a patient.

To further establish the pre-defined fluid circuit 18 (Fig. 1), an empty treated target cell container 20 is attached to an outlet of the to the fluid circuit of the cell processing device/system 12 at the time of manufacture (step 52).

Where cell processing device /system includes a reinfusion pump 40 as shown in the embodiment of Fig. 3, target cell container 20 may be preconnected to infusion pump 40. Alternatively, container 20 with target cells collected therein may be connected in a sterile manner to reinfusion pump 40 at or just prior to reinfusion.

Once the pre-defined fluid circuit 18 has been established, whole blood from whole blood collection container 14 is introduced into the cell processing device/system (step 54). The whole blood may be passively introduced into the system by, for example, gravity draining or by one or pumps that may be part of the cell processing device/system 12. The cell processing device/system 12 separates the whole blood into desired (target) cells and other components (step 56) and diverts non-targeted, unwanted blood cells and other components to a pre-connected waste container (step 57).

Continuing with the processing of the separated target cells, such cells will be treated within the cell processing device/system (step 58). As described above, treatment may involve modifying the target cells, incubating, treating with agents, treating with light etc. Once treatment is complete, the treated targeted cells are collected in the pre-connected target cell container (step 60). The treated and collected cells are then reinfused to the patient. Reinfusion preferably is carried out without the need to disconnect container 20, either by gravity infusion or by a preconnected infusion pump.

Thus, an alternative bedside cell processing system and methods of operating the system are disclosed herein. It will be appreciated that the systems and methods described herein do not involve the use or integration of apheresis systems. The description provided above, and the other aspects provided below, are intended for illustrative purposes, and are not intended to limit the scope of the disclosure to any particular apparatus, system or method described herein.

In one embodiment, the modified cells produced by the cell processing system include Chimeric Antigen Receptor T-Cells (CAR-T) cells. These CAR-T cells may be used in several applications and therapeutic treatments of various diseases, ailments, or conditions. For example, the CAR-T cells may be used to treat solid tumors such as anal/rectal, epithelial, ovarian, breast, fallopian tube, endometrial, pancreatic, colorectal, lung, and/or gastrointestinal tumors. The CAR-T cells may also be useful in therapeutically treating melanoma, multiple myeloma, acute lymphoblastic leukemia, acute myelocytic lymphoma, non-Hodgkin lymphoma, diffuse large b-cell lymphoma, small-cell lung cancer, neuroblastoma, glioblastoma, prostate cancer, hemophilia, sickle cell disease, lupus erythematosus, lupus nephritis, myasthenia gravis, autoimmune diseases, soft tissue sarcoma, osteosarcoma, hepatocellular carcinoma, graft versus host disease, and rheumatoid arthritis. The CAR-T cells may also be useful for renal transplantation patients. Accordingly, the disclosure provides a modified CAR-T cell produced by the blood processing system for use in the treatment of a disease or condition selected from the group consisting of solid tumors such as anal/rectal, epithelial, ovarian, breast, fallopian tube, endometrial, pancreatic, colorectal, lung, and/or gastrointestinal tumors; melanoma, multiple myeloma, acute lymphoblastic leukemia, acute myelocytic lymphoma, non-Hodgkin lymphoma, diffuse large b-cell lymphoma, small-cell lung cancer, neuroblastoma, glioblastoma, prostate cancer, hemophilia, sickle cell disease, lupus erythematosus, lupus nephritis, myasthenia gravis, autoimmune diseases, soft tissue sarcoma, osteosarcoma, hepatocellular carcinoma, graft versus host disease, rheumatoid arthritis, and impairments related to renal transplantation.

In producing the CAR-T cells, different modification strategies may be used in the blood processing system, with the various methods being practiced in the preparation of CAR-T cells for any one of the preceding applications or therapeutic treatments. Vectors such as mRNA, shRNA, siRNA, saRNA, SeekRNA, polymers, proteins and peptides, antibodies, viruses, including but not limited to lentivirus (LV), Adeno Associated Virus (AAV), labelling molecules, small molecules, Virus-like particles (VLP), transposon/transposase (sleeping beauty, TcBuster, PiggyBac), transcription activator-like effector nuclease (TALEN), zinc finger nucleases (ZFN), base editors, prime editors, programmable addition via site-specific targeting elements (PASTE), CRISPR/Cas, and CRISPR RNPs may be utilized in the blood processing system (specifically the cargo delivery module) to create the CAR-T cells. Accordingly, the disclosure provides in certain embodiments modified CAR-T cells produced by use of the aforementioned vectors for use in the treatment of a disease, ailment, or condition as shown in Tables 1 and 2:

**Table 1**

| **Embodiment #** | **Disease, ailment, or condition** | **Vectors for CAR-T cell modification** |
|---|---|---|
| 1 | solid tumors | mRNA, shRNA, siRNA, saRNA, SeekRNA, polymers, proteins and peptides, antibodies, viruses, including but not limited to lentivirus (LV), Adeno Associated Virus (AAV), labelling molecules, small molecules, Virus-like particles (VLP), transposon/transposase (sleeping beauty, TcBuster, PiggyBac), transcription activator-like effector nuclease (TALEN), zinc finger nucleases (ZFN), base editors, prime editors, programmable addition via site-specific targeting elements (PASTE), CRISPR/Cas, and CRISPR RNPs |
| 2 | anal tumors | mRNA, shRNA, siRNA, saRNA, SeekRNA, polymers, proteins and peptides, antibodies, viruses, including but not limited to lentivirus (LV), Adeno Associated Virus (AAV), labelling molecules, small molecules, Virus-like particles (VLP), transposon/transposase (sleeping beauty, TcBuster, PiggyBac), transcription activator-like effector nuclease (TALEN), zinc finger nucleases (ZFN), base editors, prime editors, programmable addition via site-specific targeting elements (PASTE), CRISPR/Cas, and CRISPR RNPs |
| 3 | rectal tumors | mRNA, shRNA, siRNA, saRNA, SeekRNA, polymers, proteins and peptides, antibodies, viruses, including but not limited to lentivirus (LV), Adeno Associated Virus (AAV), labelling molecules, small molecules, Virus-like particles (VLP), transposon/transposase (sleeping beauty, TcBuster, PiggyBac), transcription activator-like effector nuclease (TALEN), zinc finger nucleases (ZFN), base editors, prime editors, programmable addition via site-specific targeting elements (PASTE), CRISPR/Cas, and CRISPR RNPs |
| 4 | epithelial tumors | mRNA, shRNA, siRNA, saRNA, SeekRNA, polymers, proteins and peptides, antibodies, viruses, including but not limited to lentivirus (LV), Adeno Associated Virus (AAV), labelling molecules, small molecules, Virus-like particles (VLP), transposon/transposase (sleeping beauty, TcBuster, PiggyBac), transcription activator-like effector nuclease (TALEN), zinc finger nucleases (ZFN), base editors, prime editors, programmable addition via site-specific targeting elements (PASTE), CRISPR/Cas, and CRISPR RNPs |
| 5 | ovarian tumors | mRNA, shRNA, siRNA, saRNA, SeekRNA, polymers, proteins and peptides, antibodies, viruses, including but not limited to lentivirus (LV), Adeno Associated Virus (AAV), labelling molecules, small molecules, Virus-like particles (VLP), transposon/transposase (sleeping beauty, TcBuster, PiggyBac), transcription activator-like effector nuclease (TALEN), zinc finger nucleases (ZFN), base editors, prime editors, programmable addition via site-specific targeting elements (PASTE), CRISPR/Cas, and CRISPR RNPs |
| 6 | breast tumors | mRNA, shRNA, siRNA, saRNA, SeekRNA, polymers, proteins and peptides, antibodies, viruses, including but not limited to lentivirus (LV), Adeno Associated Virus (AAV), labelling molecules, small molecules, Virus-like particles (VLP), transposon/transposase (sleeping beauty, TcBuster, PiggyBac), transcription activator-like effector nuclease (TALEN), zinc finger nucleases (ZFN), base editors, prime editors, programmable addition via site-specific targeting elements (PASTE), CRISPR/Cas, and CRISPR RNPs |
| 7 | fallopian tube tumors | mRNA, shRNA, siRNA, saRNA, SeekRNA, polymers, proteins and peptides, antibodies, viruses, including but not limited to lentivirus (LV), Adeno Associated Virus (AAV), labelling molecules, small molecules, Virus-like particles (VLP), transposon/transposase (sleeping beauty, TcBuster, PiggyBac), transcription activator-like effector nuclease (TALEN), zinc finger nucleases (ZFN), base editors, prime editors, programmable addition via site-specific targeting elements (PASTE), CRISPR/Cas, and CRISPR RNPs |
| 8 | endometrial tumors | mRNA, shRNA, siRNA, saRNA, SeekRNA, polymers, proteins and peptides, antibodies, viruses, including but not limited to lentivirus (LV), Adeno Associated Virus (AAV), labelling molecules, small molecules, Virus-like particles (VLP), transposon/transposase (sleeping beauty, TcBuster, PiggyBac), transcription activator-like effector nuclease (TALEN), zinc finger nucleases (ZFN), base editors, prime editors, programmable addition via site-specific targeting elements (PASTE), CRISPR/Cas, and CRISPR RNPs |
| 9 | pancreatic tumors | mRNA, shRNA, siRNA, saRNA, SeekRNA, polymers, proteins and peptides, antibodies, viruses, including but not limited to lentivirus (LV), Adeno Associated Virus (AAV), labelling molecules, small molecules, Virus-like particles (VLP), transposon/transposase (sleeping beauty, TcBuster, PiggyBac), transcription activator-like effector nuclease (TALEN), zinc finger nucleases (ZFN), base editors, prime editors, programmable addition via site-specific targeting elements (PASTE), CRISPR/Cas, and CRISPR RNPs |
| 10 | colorectal tumors | mRNA, shRNA, siRNA, saRNA, SeekRNA, polymers, proteins and peptides, antibodies, viruses, including but not limited to lentivirus (LV), Adeno Associated Virus (AAV), labelling molecules, small molecules, Virus-like particles (VLP), transposon/transposase (sleeping beauty, TcBuster, PiggyBac), transcription activator-like effector nuclease (TALEN), zinc finger nucleases (ZFN), base editors, prime editors, programmable addition via site-specific targeting elements (PASTE), CRISPR/Cas, and CRISPR RNPs |
| 11 | lung tumors | mRNA, shRNA, siRNA, saRNA, SeekRNA, polymers, proteins and peptides, antibodies, viruses, including but not limited to lentivirus (LV), Adeno Associated Virus (AAV), labelling molecules, small molecules, Virus-like particles (VLP), transposon/transposase (sleeping beauty, TcBuster, PiggyBac), transcription activator-like effector nuclease (TALEN), zinc finger nucleases (ZFN), base editors, prime editors, programmable addition via site-specific targeting elements (PASTE), CRISPR/Cas, and CRISPR RNPs |
| 12 | gastrointestinal tumors | mRNA, shRNA, siRNA, saRNA, SeekRNA, polymers, proteins and peptides, antibodies, viruses, including but not limited to lentivirus (LV), Adeno Associated Virus (AAV), labelling molecules, small molecules, Virus-like particles (VLP), transposon/transposase (sleeping beauty, TcBuster, PiggyBac), transcription activator-like effector nuclease (TALEN), zinc finger nucleases (ZFN), base editors, prime editors, programmable addition via site-specific targeting elements (PASTE), CRISPR/Cas, and CRISPR RNPs |
| 13 | melanoma | mRNA, shRNA, siRNA, saRNA, SeekRNA, polymers, proteins and peptides, antibodies, viruses, including but not limited to lentivirus (LV), Adeno Associated Virus (AAV), labelling molecules, small molecules, Virus-like particles (VLP), transposon/transposase (sleeping beauty, TcBuster, PiggyBac), transcription activator-like effector nuclease (TALEN), zinc finger nucleases (ZFN), base editors, prime editors, programmable addition via site-specific targeting elements (PASTE), CRISPR/Cas, and CRISPR RNPs |
| 14 | multiple myeloma | mRNA, shRNA, siRNA, saRNA, SeekRNA, polymers, proteins and peptides, antibodies, viruses, including but not limited to lentivirus (LV), Adeno Associated Virus (AAV), labelling molecules, small molecules, Virus-like particles (VLP), transposon/transposase (sleeping beauty, TcBuster, PiggyBac), transcription activator-like effector nuclease (TALEN), zinc finger nucleases (ZFN), base editors, prime editors, programmable addition via site-specific targeting elements (PASTE), CRISPR/Cas, and CRISPR RNPs |
| 15 | acute lymphoblastic leukemia | mRNA, shRNA, siRNA, saRNA, SeekRNA, polymers, proteins and peptides, antibodies, viruses, including but not limited to lentivirus (LV), Adeno Associated Virus (AAV), labelling molecules, small molecules, Virus-like particles (VLP), transposon/transposase (sleeping beauty, TcBuster, PiggyBac), transcription activator-like effector nuclease (TALEN), zinc finger nucleases (ZFN), base editors, prime editors, programmable addition via site-specific targeting elements (PASTE), CRISPR/Cas, and CRISPR RNPs |
| 16 | acute myelocytic leukemia | mRNA, shRNA, siRNA, saRNA, SeekRNA, polymers, proteins and peptides, antibodies, viruses, including but not limited to lentivirus (LV), Adeno Associated Virus (AAV), labelling molecules, small molecules, Virus-like particles (VLP), transposon/transposase (sleeping beauty, TcBuster, PiggyBac), transcription activator-like effector nuclease (TALEN), zinc finger nucleases (ZFN), base editors, prime editors, programmable addition via site-specific targeting elements (PASTE), CRISPR/Cas, and CRISPR RNPs |
| 17 | non-Hodgkin lymphoma | mRNA, shRNA, siRNA, saRNA, SeekRNA, polymers, proteins and peptides, antibodies, viruses, including but not limited to lentivirus (LV), Adeno Associated Virus (AAV), labelling molecules, small molecules, Virus-like particles (VLP), transposon/transposase (sleeping beauty, TcBuster, PiggyBac), transcription activator-like effector nuclease (TALEN), zinc finger nucleases (ZFN), base editors, prime editors, programmable addition via site-specific targeting elements (PASTE), CRISPR/Cas, and CRISPR RNPs |
| 18 | diffuse large b-cell lymphoma | mRNA, shRNA, siRNA, saRNA, SeekRNA, polymers, proteins and peptides, antibodies, viruses, including but not limited to lentivirus (LV), Adeno Associated Virus (AAV), labelling molecules, small molecules, Virus-like particles (VLP), transposon/transposase (sleeping beauty, TcBuster, PiggyBac), transcription activator-like effector nuclease (TALEN), zinc finger nucleases (ZFN), base editors, prime editors, programmable addition via site-specific targeting elements (PASTE), CRISPR/Cas, and CRISPR RNPs |
| 19 | small-cell lung cancer | mRNA, shRNA, siRNA, saRNA, SeekRNA, polymers, proteins and peptides, antibodies, viruses, including but not limited to lentivirus (LV), Adeno Associated Virus (AAV), labelling molecules, small molecules, Virus-like particles (VLP), transposon/transposase (sleeping beauty, TcBuster, PiggyBac), transcription activator-like effector nuclease (TALEN), zinc finger nucleases (ZFN), base editors, prime editors, programmable addition via site-specific targeting elements (PASTE), CRISPR/Cas, and CRISPR RNPs |
| 20 | neuroblastoma | mRNA, shRNA, siRNA, saRNA, SeekRNA, polymers, proteins and peptides, antibodies, viruses, including but not limited to lentivirus (LV), Adeno Associated Virus (AAV), labelling molecules, small molecules, Virus-like particles (VLP), transposon/transposase (sleeping beauty, TcBuster, PiggyBac), transcription activator-like effector nuclease (TALEN), zinc finger nucleases (ZFN), base editors, prime editors, programmable addition via site-specific targeting elements (PASTE), CRISPR/Cas, and CRISPR RNPs |
| 21 | glioblastoma | mRNA, shRNA, siRNA, saRNA, SeekRNA, polymers, proteins and peptides, antibodies, viruses, including but not limited to lentivirus (LV), Adeno Associated Virus (AAV), labelling molecules, small molecules, Virus-like particles (VLP), transposon/transposase (sleeping beauty, TcBuster, PiggyBac), transcription activator-like effector nuclease (TALEN), zinc finger nucleases (ZFN), base editors, prime editors, programmable addition via site-specific targeting elements (PASTE), CRISPR/Cas, and CRISPR RNPs |
| 22 | prostate cancer | mRNA, shRNA, siRNA, saRNA, SeekRNA, polymers, proteins and peptides, antibodies, viruses, including but not limited to lentivirus (LV), Adeno Associated Virus (AAV), labelling molecules, small molecules, Virus-like particles (VLP), transposon/transposase (sleeping beauty, TcBuster, PiggyBac), transcription activator-like effector nuclease (TALEN), zinc finger nucleases (ZFN), base editors, prime editors, programmable addition via site-specific targeting elements (PASTE), CRISPR/Cas, and CRISPR RNPs |
| 23 | hemophilia | mRNA, shRNA, siRNA, saRNA, SeekRNA, polymers, proteins and peptides, antibodies, viruses, including but not limited to lentivirus (LV), Adeno Associated Virus (AAV), labelling molecules, small molecules, Virus-like particles (VLP), transposon/transposase (sleeping beauty, TcBuster, PiggyBac), transcription activator-like effector nuclease (TALEN), zinc finger nucleases (ZFN), base editors, prime editors, programmable addition via site-specific targeting elements (PASTE), CRISPR/Cas, and CRISPR RNPs |
| 24 | sickle cell disease | mRNA, shRNA, siRNA, saRNA, SeekRNA, polymers, proteins and peptides, antibodies, viruses, including but not limited to lentivirus (LV), Adeno Associated Virus (AAV), labelling molecules, small molecules, Virus-like particles (VLP), transposon/transposase (sleeping beauty, TcBuster, PiggyBac), transcription activator-like effector nuclease (TALEN), zinc finger nucleases (ZFN), base editors, prime editors, programmable addition via site-specific targeting elements (PASTE), CRISPR/Cas, and CRISPR RNPs |
| 25 | lupus erythematosus | mRNA, shRNA, siRNA, saRNA, SeekRNA, polymers, proteins and peptides, antibodies, viruses, including but not limited to lentivirus (LV), Adeno Associated Virus (AAV), labelling molecules, small molecules, Virus-like particles (VLP), transposon/transposase (sleeping beauty, TcBuster, PiggyBac), transcription activator-like effector nuclease (TALEN), zinc finger nucleases (ZFN), base editors, prime editors, programmable addition via site-specific targeting elements (PASTE), CRISPR/Cas, and CRISPR RNPs |
| 26 | lupus nephritis | mRNA, shRNA, siRNA, saRNA, SeekRNA, polymers, proteins and peptides, antibodies, viruses, including but not limited to lentivirus (LV), Adeno Associated Virus (AAV), labelling molecules, small molecules, Virus-like particles (VLP), transposon/transposase (sleeping beauty, TcBuster, PiggyBac), transcription activator-like effector nuclease (TALEN), zinc finger nucleases (ZFN), base editors, prime editors, programmable addition via site-specific targeting elements (PASTE), CRISPR/Cas, and CRISPR RNPs |
| 27 | myasthenia gravis | mRNA, shRNA, siRNA, saRNA, SeekRNA, polymers, proteins and peptides, antibodies, viruses, including but not limited to lentivirus (LV), Adeno Associated Virus (AAV), labelling molecules, small molecules, Virus-like particles (VLP), transposon/transposase (sleeping beauty, TcBuster, PiggyBac), transcription activator-like effector nuclease (TALEN), zinc finger nucleases (ZFN), base editors, prime editors, programmable addition via site-specific targeting elements (PASTE), CRISPR/Cas, and CRISPR RNPs |
| 28 | autoimmune diseases | mRNA, shRNA, siRNA, saRNA, SeekRNA, polymers, proteins and peptides, antibodies, viruses, including but not limited to lentivirus (LV), Adeno Associated Virus (AAV), labelling molecules, small molecules, Virus-like particles (VLP), transposon/transposase (sleeping beauty, TcBuster, PiggyBac), transcription activator-like effector nuclease (TALEN), zinc finger nucleases (ZFN), base editors, prime editors, programmable addition via site-specific targeting elements (PASTE), CRISPR/Cas, and CRISPR RNPs |
| 29 | soft tissue sarcoma | mRNA, shRNA, siRNA, saRNA, SeekRNA, polymers, proteins and peptides, antibodies, viruses, including but not limited to lentivirus (LV), Adeno Associated Virus (AAV), labelling molecules, small molecules, Virus-like particles (VLP), transposon/transposase (sleeping beauty, TcBuster, PiggyBac), transcription activator-like effector nuclease (TALEN), zinc finger nucleases (ZFN), base editors, prime editors, programmable addition via site-specific targeting elements (PASTE), CRISPR/Cas, and CRISPR RNPs |
| 30 | osteosarcoma | mRNA, shRNA, siRNA, saRNA, SeekRNA, polymers, proteins and peptides, antibodies, viruses, including but not limited to lentivirus (LV), Adeno Associated Virus (AAV), labelling molecules, small molecules, Virus-like particles (VLP), transposon/transposase (sleeping beauty, TcBuster, PiggyBac), transcription activator-like effector nuclease (TALEN), zinc finger nucleases (ZFN), base editors, prime editors, programmable addition via site-specific targeting elements (PASTE), CRISPR/Cas, and CRISPR RNPs |
| 31 | hepatocellular carcinoma | mRNA, shRNA, siRNA, saRNA, SeekRNA, polymers, proteins and peptides, antibodies, viruses, including but not limited to lentivirus (LV), Adeno Associated Virus (AAV), labelling molecules, small molecules, Virus-like particles (VLP), transposon/transposase (sleeping beauty, TcBuster, PiggyBac), transcription activator-like effector nuclease (TALEN), zinc finger nucleases (ZFN), base editors, prime editors, programmable addition via site-specific targeting elements (PASTE), CRISPR/Cas, and CRISPR RNPs |
| 32 | graft versus host disease | mRNA, shRNA, siRNA, saRNA, SeekRNA, polymers, proteins and peptides, antibodies, viruses, including but not limited to lentivirus (LV), Adeno Associated Virus (AAV), labelling molecules, small molecules, Virus-like particles (VLP), transposon/transposase (sleeping beauty, TcBuster, PiggyBac), transcription activator-like effector nuclease (TALEN), zinc finger nucleases (ZFN), base editors, prime editors, programmable addition via site-specific targeting elements (PASTE), CRISPR/Cas, and CRISPR RNPs |
| 33 | rheumatoid arthritis | mRNA, shRNA, siRNA, saRNA, SeekRNA, polymers, proteins and peptides, antibodies, viruses, including but not limited to lentivirus (LV), Adeno Associated Virus (AAV), labelling molecules, small molecules, Virus-like particles (VLP), transposon/transposase (sleeping beauty, TcBuster, PiggyBac), transcription activator-like effector nuclease (TALEN), zinc finger nucleases (ZFN), base editors, prime editors, programmable addition via site-specific targeting elements (PASTE), CRISPR/Cas, and CRISPR RNPs |
| 34 | impairments related to renal transplantation | mRNA, shRNA, siRNA, saRNA, SeekRNA, polymers, proteins and peptides, antibodies, viruses, including but not limited to lentivirus (LV), Adeno Associated Virus (AAV), labelling molecules, small molecules, Virus-like particles (VLP), transposon/transposase (sleeping beauty, TcBuster, PiggyBac), transcription activator-like effector nuclease (TALEN), zinc finger nucleases (ZFN), base editors, prime editors, programmable addition via site-specific targeting elements (PASTE), CRISPR/Cas, and CRISPR RNPs |

**Table 2**

| **Embodiment #** | **Vector for CAR-T cell modification** | **Diseases, ailments, or conditions** |
|---|---|---|
| 35 | mRNA | solid tumors such as anal/rectal, epithelial, ovarian, breast, fallopian tube, endometrial, pancreatic, colorectal, lung, and/or gastrointestinal tumors; melanoma, multiple myeloma, acute lymphoblastic leukemia, acute myelocytic lymphoma, non-Hodgkin lymphoma, diffuse large b-cell lymphoma, small-cell lung cancer, neuroblastoma, glioblastoma, prostate cancer, hemophilia, sickle cell disease, lupus erythematosus, lupus nephritis, myasthenia gravis, autoimmune diseases, soft tissue sarcoma, osteosarcoma, hepatocellular carcinoma, graft versus host disease, rheumatoid arthritis, and impairments related to renal transplantation |
| 36 | shRNA | solid tumors such as anal/rectal, epithelial, ovarian, breast, fallopian tube, endometrial, pancreatic, colorectal, lung, and/or gastrointestinal tumors; melanoma, multiple myeloma, acute lymphoblastic leukemia, acute myelocytic lymphoma, non-Hodgkin lymphoma, diffuse large b-cell lymphoma, small-cell lung cancer, neuroblastoma, glioblastoma, prostate cancer, hemophilia, sickle cell disease, lupus erythematosus, lupus nephritis, myasthenia gravis, autoimmune diseases, soft tissue sarcoma, osteosarcoma, hepatocellular carcinoma, graft versus host disease, rheumatoid arthritis, and impairments related to renal transplantation |
| 37 | siRNA | solid tumors such as anal/rectal, epithelial, ovarian, breast, fallopian tube, endometrial, pancreatic, colorectal, lung, and/or gastrointestinal tumors; melanoma, multiple myeloma, acute lymphoblastic leukemia, acute myelocytic lymphoma, non-Hodgkin lymphoma, diffuse large b-cell lymphoma, small-cell lung cancer, neuroblastoma, glioblastoma, prostate cancer, hemophilia, sickle cell disease, lupus erythematosus, lupus nephritis, myasthenia gravis, autoimmune diseases, soft tissue sarcoma, osteosarcoma, hepatocellular carcinoma, graft versus host disease, rheumatoid arthritis, and impairments related to renal transplantation |
| 38 | saRNA | solid tumors such as anal/rectal, epithelial, ovarian, breast, fallopian tube, endometrial, pancreatic, colorectal, lung, and/or gastrointestinal tumors; melanoma, multiple myeloma, acute lymphoblastic leukemia, acute myelocytic lymphoma, non-Hodgkin lymphoma, diffuse large b-cell lymphoma, small-cell lung cancer, neuroblastoma, glioblastoma, prostate cancer, hemophilia, sickle cell disease, lupus erythematosus, lupus nephritis, myasthenia gravis, autoimmune diseases, soft tissue sarcoma, osteosarcoma, hepatocellular carcinoma, graft versus host disease, rheumatoid arthritis, and impairments related to renal transplantation |
| 39 | SeekRNA | solid tumors such as anal/rectal, epithelial, ovarian, breast, fallopian tube, endometrial, pancreatic, colorectal, lung, and/or gastrointestinal tumors; melanoma, multiple myeloma, acute lymphoblastic leukemia, acute myelocytic lymphoma, non-Hodgkin lymphoma, diffuse large b-cell lymphoma, small-cell lung cancer, neuroblastoma, glioblastoma, prostate cancer, hemophilia, sickle cell disease, lupus erythematosus, lupus nephritis, myasthenia gravis, autoimmune diseases, soft tissue sarcoma, osteosarcoma, hepatocellular carcinoma, graft versus host disease, rheumatoid arthritis, and impairments related to renal transplantation |
| 40 | polymers | solid tumors such as anal/rectal, epithelial, ovarian, breast, fallopian tube, endometrial, pancreatic, colorectal, lung, and/or gastrointestinal tumors; melanoma, multiple myeloma, acute lymphoblastic leukemia, acute myelocytic lymphoma, non-Hodgkin lymphoma, diffuse large b-cell lymphoma, small-cell lung cancer, neuroblastoma, glioblastoma, prostate cancer, hemophilia, sickle cell disease, lupus erythematosus, lupus nephritis, myasthenia gravis, autoimmune diseases, soft tissue sarcoma, osteosarcoma, hepatocellular carcinoma, graft versus host disease, rheumatoid arthritis, and impairments related to renal transplantation |
| 41 | proteins | solid tumors such as anal/rectal, epithelial, ovarian, breast, fallopian tube, endometrial, pancreatic, colorectal, lung, and/or gastrointestinal tumors; melanoma, multiple myeloma, acute lymphoblastic leukemia, acute myelocytic lymphoma, non-Hodgkin lymphoma, diffuse large b-cell lymphoma, small-cell lung cancer, neuroblastoma, glioblastoma, prostate cancer, hemophilia, sickle cell disease, lupus erythematosus, lupus nephritis, myasthenia gravis, autoimmune diseases, soft tissue sarcoma, osteosarcoma, hepatocellular carcinoma, graft versus host disease, rheumatoid arthritis, and impairments related to renal transplantation |
| 42 | peptides | solid tumors such as anal/rectal, epithelial, ovarian, breast, fallopian tube, endometrial, pancreatic, colorectal, lung, and/or gastrointestinal tumors; melanoma, multiple myeloma, acute lymphoblastic leukemia, acute myelocytic lymphoma, non-Hodgkin lymphoma, diffuse large b-cell lymphoma, small-cell lung cancer, neuroblastoma, glioblastoma, prostate cancer, hemophilia, sickle cell disease, lupus erythematosus, lupus nephritis, myasthenia gravis, autoimmune diseases, soft tissue sarcoma, osteosarcoma, hepatocellular carcinoma, graft versus host disease, rheumatoid arthritis, and impairments related to renal transplantation |
| 43 | antibodies | solid tumors such as anal/rectal, epithelial, ovarian, breast, fallopian tube, endometrial, pancreatic, colorectal, lung, and/or gastrointestinal tumors; melanoma, multiple myeloma, acute lymphoblastic leukemia, acute myelocytic lymphoma, non-Hodgkin lymphoma, diffuse large b-cell lymphoma, small-cell lung cancer, neuroblastoma, glioblastoma, prostate cancer, hemophilia, sickle cell disease, lupus erythematosus, lupus nephritis, myasthenia gravis, autoimmune diseases, soft tissue sarcoma, osteosarcoma, hepatocellular carcinoma, graft versus host disease, rheumatoid arthritis, and impairments related to renal transplantation |
| 44 | viruses | solid tumors such as anal/rectal, epithelial, ovarian, breast, fallopian tube, endometrial, pancreatic, colorectal, lung, and/or gastrointestinal tumors; melanoma, multiple myeloma, acute lymphoblastic leukemia, acute myelocytic lymphoma, non-Hodgkin lymphoma, diffuse large b-cell lymphoma, small-cell lung cancer, neuroblastoma, glioblastoma, prostate cancer, hemophilia, sickle cell disease, lupus erythematosus, lupus nephritis, myasthenia gravis, autoimmune diseases, soft tissue sarcoma, osteosarcoma, hepatocellular carcinoma, graft versus host disease, rheumatoid arthritis, and impairments related to renal transplantation |
| 45 | Lentivirus (LV) | solid tumors such as anal/rectal, epithelial, ovarian, breast, fallopian tube, endometrial, pancreatic, colorectal, lung, and/or gastrointestinal tumors; melanoma, multiple myeloma, acute lymphoblastic leukemia, acute myelocytic lymphoma, non-Hodgkin lymphoma, diffuse large b-cell lymphoma, small-cell lung cancer, neuroblastoma, glioblastoma, prostate cancer, hemophilia, sickle cell disease, lupus erythematosus, lupus nephritis, myasthenia gravis, autoimmune diseases, soft tissue sarcoma, osteosarcoma, hepatocellular carcinoma, graft versus host disease, rheumatoid arthritis, and impairments related to renal transplantation |
| 46 | Adeno Associated Virus (AAV) | solid tumors such as anal/rectal, epithelial, ovarian, breast, fallopian tube, endometrial, pancreatic, colorectal, lung, and/or gastrointestinal tumors; melanoma, multiple myeloma, acute lymphoblastic leukemia, acute myelocytic lymphoma, non-Hodgkin lymphoma, diffuse large b-cell lymphoma, small-cell lung cancer, neuroblastoma, glioblastoma, prostate cancer, hemophilia, sickle cell disease, lupus erythematosus, lupus nephritis, myasthenia gravis, autoimmune diseases, soft tissue sarcoma, osteosarcoma, hepatocellular carcinoma, graft versus host disease, rheumatoid arthritis, and impairments related to renal transplantation |
| 47 | labelling molecules | solid tumors such as anal/rectal, epithelial, ovarian, breast, fallopian tube, endometrial, pancreatic, colorectal, lung, and/or gastrointestinal tumors; melanoma, multiple myeloma, acute lymphoblastic leukemia, acute myelocytic lymphoma, non-Hodgkin lymphoma, diffuse large b-cell lymphoma, small-cell lung cancer, neuroblastoma, glioblastoma, prostate cancer, hemophilia, sickle cell disease, lupus erythematosus, lupus nephritis, myasthenia gravis, autoimmune diseases, soft tissue sarcoma, osteosarcoma, hepatocellular carcinoma, graft versus host disease, rheumatoid arthritis, and impairments related to renal transplantation |
| 48 | small molecules | solid tumors such as anal/rectal, epithelial, ovarian, breast, fallopian tube, endometrial, pancreatic, colorectal, lung, and/or gastrointestinal tumors; melanoma, multiple myeloma, acute lymphoblastic leukemia, acute myelocytic lymphoma, non-Hodgkin lymphoma, diffuse large b-cell lymphoma, small-cell lung cancer, neuroblastoma, glioblastoma, prostate cancer, hemophilia, sickle cell disease, lupus erythematosus, lupus nephritis, myasthenia gravis, autoimmune diseases, soft tissue sarcoma, osteosarcoma, hepatocellular carcinoma, graft versus host disease, rheumatoid arthritis, and impairments related to renal transplantation |
| 49 | Virus-like particles (VLP) | solid tumors such as anal/rectal, epithelial, ovarian, breast, fallopian tube, endometrial, pancreatic, colorectal, lung, and/or gastrointestinal tumors; melanoma, multiple myeloma, acute lymphoblastic leukemia, acute myelocytic lymphoma, non-Hodgkin lymphoma, diffuse large b-cell lymphoma, small-cell lung cancer, neuroblastoma, glioblastoma, prostate cancer, hemophilia, sickle cell disease, lupus erythematosus, lupus nephritis, myasthenia gravis, autoimmune diseases, soft tissue sarcoma, osteosarcoma, hepatocellular carcinoma, graft versus host disease, rheumatoid arthritis, and impairments related to renal transplantation |
| 50 | transposon/transposase (sleeping beauty) | solid tumors such as anal/rectal, epithelial, ovarian, breast, fallopian tube, endometrial, pancreatic, colorectal, lung, and/or gastrointestinal tumors; melanoma, multiple myeloma, acute lymphoblastic leukemia, acute myelocytic lymphoma, non-Hodgkin lymphoma, diffuse large b-cell lymphoma, small-cell lung cancer, neuroblastoma, glioblastoma, prostate cancer, hemophilia, sickle cell disease, lupus erythematosus, lupus nephritis, myasthenia gravis, autoimmune diseases, soft tissue sarcoma, osteosarcoma, hepatocellular carcinoma, graft versus host disease, rheumatoid arthritis, and impairments related to renal transplantation |
| 51 | transposon/transposase (TcBuster) | solid tumors such as anal/rectal, epithelial, ovarian, breast, fallopian tube, endometrial, pancreatic, colorectal, lung, and/or gastrointestinal tumors; melanoma, multiple myeloma, acute lymphoblastic leukemia, acute myelocytic lymphoma, non-Hodgkin lymphoma, diffuse large b-cell lymphoma, small-cell lung cancer, neuroblastoma, glioblastoma, prostate cancer, hemophilia, sickle cell disease, lupus erythematosus, lupus nephritis, myasthenia gravis, autoimmune diseases, soft tissue sarcoma, osteosarcoma, hepatocellular carcinoma, graft versus host disease, rheumatoid arthritis, and impairments related to renal transplantation |
| 52 | transposon/transposase (PiggyBac) | solid tumors such as anal/rectal, epithelial, ovarian, breast, fallopian tube, endometrial, pancreatic, colorectal, lung, and/or gastrointestinal tumors; melanoma, multiple myeloma, acute lymphoblastic leukemia, acute myelocytic lymphoma, non-Hodgkin lymphoma, diffuse large b-cell lymphoma, small-cell lung cancer, neuroblastoma, glioblastoma, prostate cancer, hemophilia, sickle cell disease, lupus erythematosus, lupus nephritis, myasthenia gravis, autoimmune diseases, soft tissue sarcoma, osteosarcoma, hepatocellular carcinoma, graft versus host disease, rheumatoid arthritis, and impairments related to renal transplantation |
| 53 | transcription activator-like effector nuclease (TALEN) | solid tumors such as anal/rectal, epithelial, ovarian, breast, fallopian tube, endometrial, pancreatic, colorectal, lung, and/or gastrointestinal tumors; melanoma, multiple myeloma, acute lymphoblastic leukemia, acute myelocytic lymphoma, non-Hodgkin lymphoma, diffuse large b-cell lymphoma, small-cell lung cancer, neuroblastoma, glioblastoma, prostate cancer, hemophilia, sickle cell disease, lupus erythematosus, lupus nephritis, myasthenia gravis, autoimmune diseases, soft tissue sarcoma, osteosarcoma, hepatocellular carcinoma, graft versus host disease, rheumatoid arthritis, and impairments related to renal transplantation |
| 54 | zinc finger nucleases (ZFN) | solid tumors such as anal/rectal, epithelial, ovarian, breast, fallopian tube, endometrial, pancreatic, colorectal, lung, and/or gastrointestinal tumors; melanoma, multiple myeloma, acute lymphoblastic leukemia, acute myelocytic lymphoma, non-Hodgkin lymphoma, diffuse large b-cell lymphoma, small-cell lung cancer, neuroblastoma, glioblastoma, prostate cancer, hemophilia, sickle cell disease, lupus erythematosus, lupus nephritis, myasthenia gravis, autoimmune diseases, soft tissue sarcoma, osteosarcoma, hepatocellular carcinoma, graft versus host disease, rheumatoid arthritis, and impairments related to renal transplantation |
| 55 | base editors | solid tumors such as anal/rectal, epithelial, ovarian, breast, fallopian tube, endometrial, pancreatic, colorectal, lung, and/or gastrointestinal tumors; melanoma, multiple myeloma, acute lymphoblastic leukemia, acute myelocytic lymphoma, non-Hodgkin lymphoma, diffuse large b-cell lymphoma, small-cell lung cancer, neuroblastoma, glioblastoma, prostate cancer, hemophilia, sickle cell disease, lupus erythematosus, lupus nephritis, myasthenia gravis, autoimmune diseases, soft tissue sarcoma, osteosarcoma, hepatocellular carcinoma, graft versus host disease, rheumatoid arthritis, and impairments related to renal transplantation |
| 56 | prime editors | solid tumors such as anal/rectal, epithelial, ovarian, breast, fallopian tube, endometrial, pancreatic, colorectal, lung, and/or gastrointestinal tumors; melanoma, multiple myeloma, acute lymphoblastic leukemia, acute myelocytic lymphoma, non-Hodgkin lymphoma, diffuse large b-cell lymphoma, small-cell lung cancer, neuroblastoma, glioblastoma, prostate cancer, hemophilia, sickle cell disease, lupus erythematosus, lupus nephritis, myasthenia gravis, autoimmune diseases, soft tissue sarcoma, osteosarcoma, hepatocellular carcinoma, graft versus host disease, rheumatoid arthritis, and impairments related to renal transplantation |
| 57 | programmable addition via site-specific targeting elements (PASTE) | solid tumors such as anal/rectal, epithelial, ovarian, breast, fallopian tube, endometrial, pancreatic, colorectal, lung, and/or gastrointestinal tumors; melanoma, multiple myeloma, acute lymphoblastic leukemia, acute myelocytic lymphoma, non-Hodgkin lymphoma, diffuse large b-cell lymphoma, small-cell lung cancer, neuroblastoma, glioblastoma, prostate cancer, hemophilia, sickle cell disease, lupus erythematosus, lupus nephritis, myasthenia gravis, autoimmune diseases, soft tissue sarcoma, osteosarcoma, hepatocellular carcinoma, graft versus host disease, rheumatoid arthritis, and impairments related to renal transplantation |
| 58 | CRISPR/Cas | solid tumors such as anal/rectal, epithelial, ovarian, breast, fallopian tube, endometrial, pancreatic, colorectal, lung, and/or gastrointestinal tumors; melanoma, multiple myeloma, acute lymphoblastic leukemia, acute myelocytic lymphoma, non-Hodgkin lymphoma, diffuse large b-cell lymphoma, small-cell lung cancer, neuroblastoma, glioblastoma, prostate cancer, hemophilia, sickle cell disease, lupus erythematosus, lupus nephritis, myasthenia gravis, autoimmune diseases, soft tissue sarcoma, osteosarcoma, hepatocellular carcinoma, graft versus host disease, rheumatoid arthritis, and impairments related to renal transplantation |
| 59 | CRISPR RNPs | solid tumors such as anal/rectal, epithelial, ovarian, breast, fallopian tube, endometrial, pancreatic, colorectal, lung, and/or gastrointestinal tumors; melanoma, multiple myeloma, acute lymphoblastic leukemia, acute myelocytic lymphoma, non-Hodgkin lymphoma, diffuse large b-cell lymphoma, small-cell lung cancer, neuroblastoma, glioblastoma, prostate cancer, hemophilia, sickle cell disease, lupus erythematosus, lupus nephritis, myasthenia gravis, autoimmune diseases, soft tissue sarcoma, osteosarcoma, hepatocellular carcinoma, graft versus host disease, rheumatoid arthritis, and impairments related to renal transplantation |

Embodiments 1 to 59 listed in Tables 1 and 2 do not only apply to modified CAR-T cells, but also to any other modified cell types that can be produced by the blood processing system.

Accordingly, a "modified cell" as used herein is a cell that has been produced by the cell processing system by use of the vectors of embodiments 35 to 59 (Table 2). Said modified cell is further provided for use in the treatment of a disease, ailment, or condition according to embodiments 1 to 34 (Table 1).

In an embodiment, the modified cells are modified Chimeric Antigen Receptor Natural Killer (CAR-NK) cells.

In a further embodiment, the modified cells are modified Chimeric Antigen Receptor Monocytes (CAR-M) cells.

In another embodiment, the modified cells are modified Engineered T-Cell Receptor (TCR) cells.

In a further embodiment, the modified cells are modified Engineered B-Cells.

In another embodiment, the modified cells are modified Tumor Infiltrating Lymphocytes (TIL) cells.

In a further embodiment, the modified cells are modified Induced Pluripotent Stem (iPSC) Cells.

In another embodiment, the modified cells are modified Invariant Natural Killer T (iNKT) cells

In a further embodiment, the modified cells are modified Mesenchymal Stem (MSC) cells.

In another embodiment, the modified cells are modified Dendritic Cells.

In a further embodiment, the modified cells are modified Hematopoietic Stem Cells/Stem (CD34+) cells.

The modified cells can be produced and administered in a variety of environments by the blood processing system, with the modified cells being produced using any one of the preceding vectors and being administered in any one of the preceding applications or therapeutic treatments. By way of example, the blood processing system can produce and/or administer modified cells within a patient treatment room/facility (inpatient or outpatient). The blood processing system can produce and/or administer modified cells within a cell processing lab. The cell processing lab may be at a hospital facility, at a non-hospital facility, or a commercial production facility (centralized or decentralized). The blood processing system can produce and/or administer modified cells within a sterile manufacturing suite. The sterile manufacturing suite may be at a hospital facility, at a non-hospital facility, at an academic facility, or at commercial production facility (centralized or decentralized).

While the modified cells produced by this system are described as being used to treat particular conditions and patient groups, it should be understood that they may be applied to other conditions and/or patient groups, including sub-groups of a described patient group (i.e., patients having the same characteristics that characterize a particular patient group, but additional characteristics that are not shared by all patients of that patient group), larger patient groups encompassing a described patient group (i.e., a patient group having broadly defined characteristics that include the characteristics that characterize a particular patient group) and entirely different patient groups (i.e., patients having characteristics that exclude them from a particular patient group). The modified cells may also be applied in various dosages, administrative regimes, and routes of administration without departing from the scope of the present disclosure.

It should be understood that the embodiments disclosed herein may be combined with each other in any imaginable combination.

There are additional aspects to the systems, devices and methods described herein including, without limitation the following Aspects.

Aspect 1. A bedside cell processing system, including a source of previously collected whole blood; a cell processing device configured to receive the target cells from the whole blood source and to separate target cells from the whole blood and further process said target cells; and a reinfusion system configured to receive the processed target cells from the cell processing system and to return the processed cells to a patient; and a pre-defined fluid pathway providing flow communication between said whole blood source and said patient.

Aspect 2. The bedside cell processing system of Aspect 1 wherein said source of previously collected whole blood includes a container preconnected to said cell processing system.

Aspect 3. The bedside cell processing system of any one of Aspects 1 and 2 wherein said reinfusion system includes a container pre-connected to said cell processing system.

Aspect 4. The bedside cell processing system of any one of Aspects 1 through 3 including a waste container (pre)connected to the cell processing system.

Aspect 5. The bedside cell processing system of any one of Aspects 1 through 4 wherein said reinfusion system includes a preconnected reinfusion container and a preconnected infusion pump.

Aspect 6. The bedside cell processing system of Aspect 5 wherein said reinfusion pump is connected to a reinfusion container that is preconnected to said cell processing device.

Aspect 7. The bedside cell processing system of any one of Aspects 5 and 6 wherein said bedside cell processing system includes a controller configured to initiate reinfusion of treated target cells from said reinfusion system to a patient.

Aspect 8. The bedside cell processing system of Aspect 7 wherein said controller is associated with said reinfusion pump.

Aspect 9. The bedside cell processing system of Aspect 1 including sterile connection means for pre-connecting a container of whole blood to said cell processing device.

Aspect 10. The bedside cell processing system of Aspect 1 including sterile connection means for connecting a container of treated target cells to said reinfusion system.

Aspect 11. A blood processing method including connecting a container for receiving whole blood to an inlet of a cell processing device; connecting a treated target cell product container to an outlet of said cell processing device; introducing whole blood from said container for receiving whole blood into said cell processing device; separating said whole blood into target cells and other blood components in said cell processing device; treating said target cells within said cell processing device; introducing said treated target cells into said target cell product container; infusing said treated target cells from said target cell product container to a patient.

Aspect 12. The method of Aspect 11 including collecting whole blood from a patient in said container for receiving whole blood prior to connecting said whole blood receiving container to said cell processing device.

Aspect 13. The method of any one of Aspects 11 and 12 including diverting said other components to a waste container connected to said cell processing device.

Aspect 14. The method of any one of Aspects 11 through 13 including infusing said treated target cells to a patient.

Aspect 15. The method of Aspect 14 including pumping said treated target cells from said treated target cell product container to said patient.

Aspect 16. The method of Aspect 15 including sterilely connecting said treated target cell product container to an infusion pump.

Aspect 17. The method of any one of Aspects 15 and 16 including initiating infusion of said treated target cells to said patient when a pre-selected volume of treated target cells has been collected.

Aspect 18. The method of any one of Aspects 14 through 17 including infusing autologous cells of said patient.

It will be understood that the embodiments and examples described above are illustrative of some of the applications or principles of the present subject matter. Numerous modifications may be made by those skilled in the art without departing from the spirit and scope of the claimed subject matter, including those combinations of features that are individually disclosed or claimed herein. For these reasons, the scope hereof is not limited to the above description but is as set forth in the following claims, and it is understood that the claims may be directed to the features thereof, including as combinations of features that are individually disclosed or claimed herein.

## Claims

1. A bedside cell processing system, comprising:
a source of previously collected whole blood;
a cell processing device configured to receive the target cells from the whole blood source and to separate target cells from the whole blood and
further process said target cells; and a reinfusion system configured to receive the processed target cells from the cell processing system and to return the processed cells to a patient; and a pre-defined fluid pathway providing flow communication between said whole blood source and said patient.

2. The bedside cell processing system of Claim 1 wherein said source of previously collected whole blood comprises a container preconnected to said cell processing system.

3. The bedside cell processing system of any one of Claims 1 and 2 wherein said reinfusion system comprises a container pre-connected to said cell processing system.

4. The bedside cell processing system of any one of Claims 1 through 3 comprising a waste container (pre)connected to the cell processing system.

5. The bedside cell processing system of any one of Claims 1 through 4 wherein said reinfusion system comprises a preconnected reinfusion container and a preconnected infusion pump.

6. The bedside cell processing system of Claim 5 wherein said reinfusion pump is connected to a reinfusion container that is preconnected to said cell processing device.

7. The bedside cell processing system of any one of Claims 5 and 6 wherein said bedside cell processing system comprises a controller configured to initiate reinfusion of treated target cells from said reinfusion system to a patient.

8. The bedside cell processing system of Claim 7 wherein said controller is associated with said reinfusion pump.

9. The bedside cell processing system of Claim 1 comprising sterile connection means for pre-connecting a container of whole blood to said cell processing device.

10. The bedside cell processing system of Claim 1 comprising sterile connection means for connecting a container of treated target cells to said reinfusion system.

11. A blood processing method comprising:
connecting a container for receiving whole blood to an inlet of a cell processing device;
connecting a treated target cell product container to an outlet of said cell processing device;
introducing whole blood from said container for receiving whole blood into said cell processing device;
separating said whole blood into target cells and other blood components in said cell processing device;
treating said target cells within said cell processing device; and
introducing said treated target cells into said target cell product container.

12. The method of Claim 11 comprising diverting said other components to a waste container connected to said cell processing device.

13. The method of any of Claims 11 and 12 comprising sterilely connecting said treated target cell product container to an infusion pump.

14. The method of any one of Claims 11 through 13 wherein said target cells are autologous cells.
